# EUROPEAN PATENT SPECIFICATION

(11) **EP 0 657 456 B1**
(45) Date of publication and mention of the grant of the patent: **04.06.1997**
(21) Application number: 94119544.8
(22) Date of filing: 09.12.1994
(51) Int. Cl.: C07D 487/08, A61K 31/44

(54) **Process for preparing epibatidine**
Verfahren zur Herstellung von Epibatidin
Procédé pour la préparation d'épibatidine

(30) Priority: 09.12.1993 HU 3500593; 06.10.1994 HU 3500593
(43) Date of publication of application: 14.06.1995
(60) Divisional application: 96107729.4
(73) Proprietor: EGIS GYOGYSZERGYAR RT., H-1106 Budapest (HU)
(72) Inventor: Csaba Szantay Dr., chemical engineer, H-1113 Budapest (HU); Zsuzsanna Balogh née Kardos, Dr., chemist, H-1125 Budapest (HU); Istvan Moldvai, Dr., chemistry teacher, H-1212 Budapest (HU); Eszter Temesvari née Major, Dr., chemical engineer, H-1116 Budapest (HU); Csaba Szantay jr., Dr., chemical engineer, H-1113 Budapest (HU); Attila Mandi, Dr., chemical engineer, H-1026 Budapest (HU); Gabor Blasko, Dr., chemical engineer, H-1113 Budapest (HU); Gyula Simig, Dr., chemical engineer, H-1126 Budapest (HU); Sandor Drabant, Dr., physician, H-1171 Budapest (HU); Tamas Szallasi, Dr., physician, H-1027 Budapest (HU); Marton Fekete, Dr., physician, H-1027 Budapest (HU); Gabor Gigler, biologist, H-1067 Budapest (HU)
(74) Representative: Beszédes, Stephan G., Dr.

(56) References cited:
- WO-A-93/18037
- JOURNAL OF THE CHEMICAL SOCIETY, CHEMICAL COMMUNICATIONS, no.15, 7 August 1993, LETCHWORTH GB pages 1216 - 1218 S.R. FLETCHER ET AL. 'The synthesis of (+)- and (-)-epibatidine'
- JOURNAL OF THE CHEMICAL SOCIETY, CHEMICAL COMMUNICATIONS, no.15, 17 August 1994, LETCHWORTH GB S.Y. KO ET AL. 'The total synthesis of epibatidine'
- TETRAHEDRON LETTERS., vol.35, no.19, 9 May 1994, OXFORD GB pages 3171 - 3174 C. SZANTAY ET AL. 'A practical route to epibatidine'
- TETRAHEDRON LETTERS., vol.34, no.28, 1993, OXFORD GB pages 4477 - 4480 D.F. HUANG ET AL. 'A versatile total synthesis of epibatidine and analogs'
- JOURNAL OF ORGANIC CHEMISTRY., vol.58, no.21, 1993, EASTON US pages 5600 - 5602 E.J. COREY ET AL. 'Stereocontrolled total synthesis of (+)- and (-)-epibatidine'

## Description

The invention is concerned with a process for preparing an organic compound, namely epibatidine, i. e. 2-[6'-(chloro)-pyrid-3'-yl]-7-azabicyclo[2.2.1]heptane, of formula 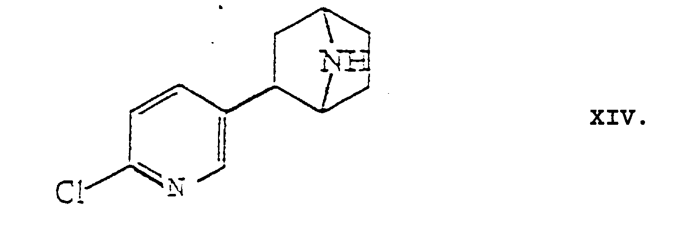 Epibatidine is a minor alkaloid isolated from the skin extracts of an Ecuadoran frog, Epipedobates tricolor, of the family Dendrobatidae. Considering the chemical structure it is the first natural substance containing a 7-azabicyclo[2.2.1]heptane skeleton. To the exo position of the said skeleton a 2-(chloro)-pyrid-3-yl substituent is attached, which can rarely be found in nature. The alkaloid possesses a valuable biological property, namely it has 200 times the analgesic potency of morphine. The mechanism of the analgesic effect is different from that of morphine, as it does not subside upon administering morphine antagonists, e.g. 17--(allyl)-4,5α-(epoxy)-3,14-di-(hydroxy)-morphinan-6--one [naloxone] (T. F. Spande, H. M. Garraffo, M. W. Edwards, H. J. C. Yeh, L. Pannell, J. W. Daly: J. Am. Chem. Soc., 1992, 114, 3 475 to 3 478).

Several processes have been disclosed in the literature for the synthetic preparation of epibatidine:
A) Ch. A. Broka: Tetrahedron Letters, 34 [1993], 3 251 to 3 254. In this reaction an adduct was prepared by the Diels-Alder reaction of enal obtained by the homologization of 6-(chloro)-nicotinic aldehyde and 2-(trimethylsilyloxy)-1,3-butadiene, it was then reduced with lithium, sodium and potassium tri-2-butyl borohydride [L--selectride®], the hydroxy group of the product was protected by silylation, while the hydroxymethyl substituent was first converted into the hydroxy group in 6 reaction steps and then benzoylated. By selective removal of the protecting groups the dihydroxy derivative was converted into 4-(mesyloxy)-cyclohexylamine containing a 6-(chloro)-pyridyl substituent, and the boiling of the latter compound in dichloromethane resulted in racemic epibatidine.
B) J. W. Daly, T.F. Spande, H. M. Garraffo: US Dept. Health and Human Service, WO 93/18037. The key intermediate of this synthesis is 3-(pyridyl)-2-cyclohexa-1,3-diene, which was prepared from cyclohexane-1,2-dione and converted into Diels-Alder adduct with tert.butylnitrosoformiate. The adduct was hydrogenated catalytically, the thus-obtained aminoalcohol derivative the amino group of which is protected by a tert.-butylformate group was treated with thionyl chloride, the product, the corresponding 1-[pyrid-3'-yl]-2-[chloro]-5-[amino]-cyclohexane protected on the nitrogen atom of the amino group by a tert.-butylformate group, was cyclized with base-treatment to give 1-[pyrid-3'-yl]-7-azabicyclo[2.2.1]heptane protected on its aza-nitrogen atom by a tert.-butylformate group and chlorinated by a photochemical reaction to 1-[pyrid-3'-yl]-7-azabicyclo[2.2.1]heptane protected on its aza-nitrogen atom by a tert.-butylformate group. The hydrolysis of the acid amide bond with trifluoroacetic acid resulted in racemic epibatidine.
C) D. F. Huang, T. Y. Shen: Tetrahedron Letters, 34 [1993], 4 477 to 4 480. An adduct obtained by the Diels-Alder reaction of phenylsulfonyl-6-(chloro)-3-(pyridyl)-acetylene prepared from N-(carbomethoxy)-pyrrole and 6-(chloro)-nicotinic acid was desulfonated with sodium amalgam, concomitantly reducing one of the double bonds of the 7-azanorbornadiene ring, hydrogenated catalytically to a mixture of N-(carbomethoxy)-epi-epibatidine and N-(carbomethoxy)-epibatidine, the protecting group was dehydrolized to obtain racemic epibatidine and racemic epi-epibatidine, which were then separated by column chromatography. The racemic epibatidine was resolved with di- (p-toluyl)-tartaric acid.
D) S. R. Fletcher, R. Baker, M. S. Chambers, S. C. Hobbs, P. J. Mitchell: J. Chem. Soc. Chem. Comm., 1993, 1 216 to 1 218. The key intermediate of this synthesis route is tert.butyloxycarbonyl-7-azabicyclo[2.2.1]heptan-2-one, which was prepared in 7 reaction steps from cyclohexene amine containing a trifluoroacetyl protecting group and reacted with 5-lithio-2-(chloro)-pyridine. From the thus-obtained adduct water was removed and the thus-formed double bond was hydrogenated catalytically to obtain a mixture of racemic epibatidine and epi-epibatidine isomers containing tert.butyloxycarbonyl protecting group. The undesired epimer epi-epibatidine (endo-isomer) protected on the nitrogen atom of the 7-azabicyclo[2.2.1]heptane ring by a tert.-butoxycarbonyl protective group (BOC protective group) was subjected to epimerization by boiling in tert.butanol in the presence of potassium tert.butylate. The racemic epibatidine (exo-isomer) was resolved by applying a chiral HPLC method and by salt formation with a chiral acid.
E) E. J. Corey,* Teck-Peng Loh, Sidduri AchyuthaRao, Donnette C. Daley, and Sepehr Sarshar: Journal of Organic Chemistry, Vol. 58, No. 21, 1993, 5 600 to 5 602. From this it has been known to cyclize 1-[trifluoracetylamino]-2-[6'-(chloro)-pyrid-3'-yl]-4,5-di[bromo]-cyclohexane cyclizing with potassium tert.-butyloxyd in tetrahydrofurane to dextro 5-[bromo]-N-[trifluoroacetyl]-epibatidine, to debrominate the latter to dextro N-(trifluoroacetyl)-epibatidine by treatment with tributyltin and aluminumbornitride in benzene and to deacylate the latter with sodium methylate in methanol to yield (-)-epibatidine.

A serious drawback of the methods known from the literature resides in the cumbersome reaction steps which would make the industrial scale application rather complicated.

A further disadvantage of the known preparation methods resides in the fact that they require the application of difficultly available, expensive and inconvenient substances.

The problem underlying to the invention is to create a novel process for preparing epibatidine, which is devoid of the drawbacks of the hitherto known processes and can be accomplished by using substances readily available and preparable even on an industrial scale and which is also industrially applicable.

The above surprisingly has been attained by the invention.

The subject matter of the invention is a process for preparing racemic or optically active epibatidine of formula 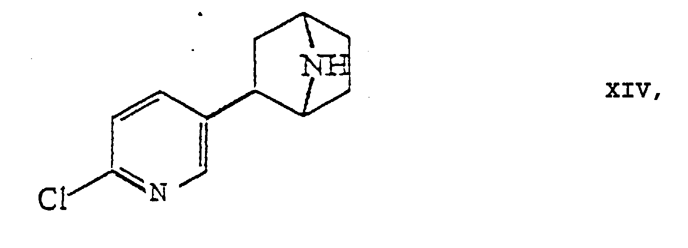 characterized by
a)
   α) cyclising a racemic or optically active 1-[amino]-2-[6'-(chloro)-pyrid-3'-yl]-cyclohexane, 4-substituted by a leaving group, of general formula 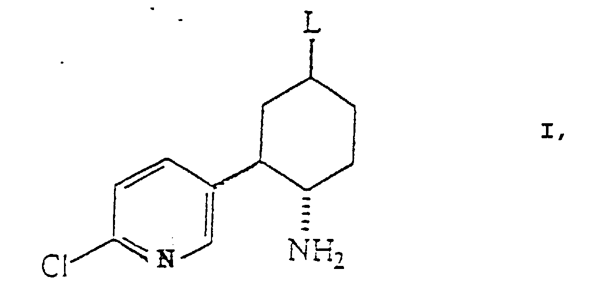 wherein
      - L: stands for a leaving group, particularly a C₁₋₄-alkylsulfonyloxy or arylsulfonyloxy group, to racemic or optically active epi-epibatidine of formula 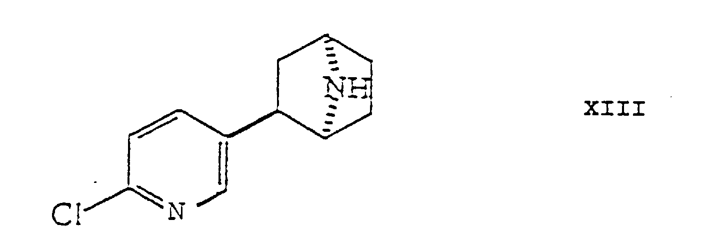 and thereafter
   β) subjecting the latter to epimerization in the presence of a base
      or
b) cyclizing 1-[6'-(chloro)-pyrid-3'-yl]-3-[oxo]-6-(nitro]-hexa-1-ene of formula 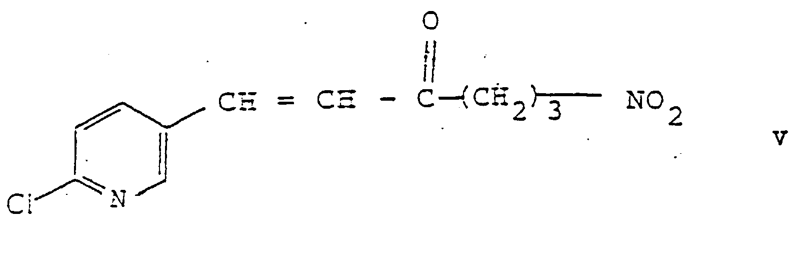 in the presence of an optically active base to optically active 1-[nitro]-2-[6'-(chloro)-pyrid-3'-yl]-cyclohexane-4-one of formula IV
   and thereafter
   α) selectively reducing the optically active 1-[nitro]-2-[6'-(chloro)-pyrid-3'-yl]-cyclohexane-4-one of formula IV thus obtained to yield optically active 1-[nitro]-2-[6'-(chloro)-pyrid-3'-yl]-cyclohexane-4-ol of formula 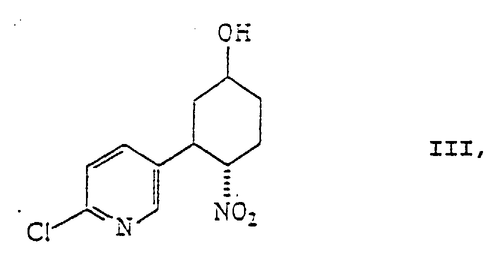
   β) introducing into the latter a leaving group to yield an optically active 1-[nitro]-2-[6'-(chloro)-pyrid-3'-yl]-cyclohexane, 4-substituted by a leaving group, of general formula 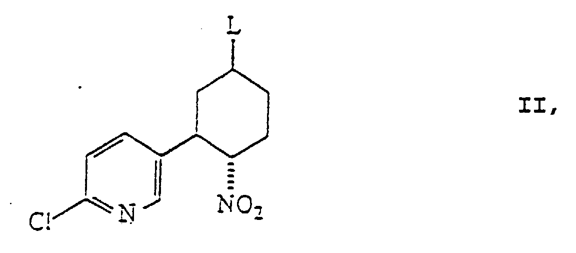 wherein
      - L: represents a leaving group, particularly a C₁₋₄-alkylsulfonyloxy or arylsulfonyloxy group,
   γ) reducing the latter to an optically active 1-[amino]-2 -[6'-(chloro)-pyrid-3'-yl]-cyclohexane, 4-substituted by a leaving group, of general formula 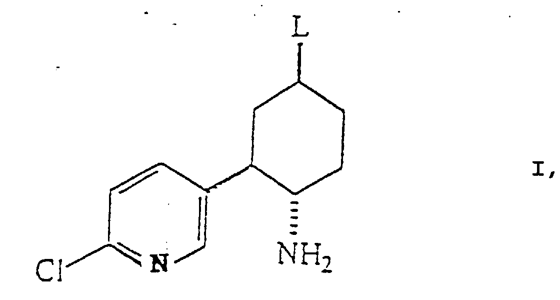
   δ) cyclising the latter to an optically active epi-epibatidine of formula XIII
      and
   ε) subjecting the latter to epimerization in the presence of a base.

If desired in a manner known per se a thus obtained racemic epibatidine of formula XIV may be resolved into the optically active isomers and/or the thus obtained optically active isomers may be racemised or, if desired, a racemic intermediate product may be separated in any stage of the synthesis into the optically active isomers and the thus obtained optically active isomers may be converted into optically active epibatidine of formula XIV.

As opposed to WO 93/18037 in which for the cyclisation an amino group which is protected by a tert.-butylformate group is present and no chlorine substituent on the pyridine ring in steps a)α) and b)δ) the cyclisation is carried out with a compound in which the amino group is not protected and on the pyridine ring already the chlorine substituent of epibatidine is present.

Analogous is the situation as compared to Journal of Organic Chemistry, Vol. 58, No. 21, 1993, pages 5 600 to 5 602 with the modification that there the protective group on the nitrogen atom of the amino group is a trifluoroacetyl group.

Preferably the epimerization of the epi-epibatidine of formula XIII according to variant a), part β) of the process according to the invention is carried out under heating, particularly at the boiling point of the reaction mixture. Preferably using for the epimerization an alkali alcoholate, particularly potassium tert.butylate or sodium ethylate, or another organic alkali compound, particularly a lithium salt of a secondary amine, above all lithium diisopropyl amine, is used. A further example of another organic alkali compound is butyl lithium. Advantageously the epimerization in the presence of an alkali alcoholate is carried out under heating and the epimerization in the presence of an other organic alkali compound at a temperature of about 0°C. It is preferable to carry out the epimerization in a C₁₋₄-alkanol as medium in the presence of an alkali alcoholate corresponding to the alkanol used as solvent.

The epibatidine of formula XIV has a chiral structure so that it can be present in racemic or optically active form. The invention encompasses the preparation of both the racemic and the optically active epibatidine of formula XIV.

The racemic epibatidine of formula XIV can optionally be resolved. The resolution can be carried out by methods known per se. One can proceed e. g. by reacting the racemic epibatidine of general formula XIV with an optically active acid, such as tartaric acid, di -(0,0'-p-toluyl)-tartaric acid or dibenzoyltartaric acid, separating the thus-obtained diastereoisomeric salt pair, e. g. by fractional crystallization and liberating the desired optically active epibatidine of formula XIV from the salt thereof.

In variant a), part α) of the process according to the invention preferably a 1-[amino]-2-[6'-(chloro)-pyrid-3'-yl]-cyclohexane, 4-substituted by a leaving group, of general formula I, in which the leaving group represented by L is a methanesulfonyloxy, p-toluenesulfonyloxy or p-(bromo)-phenylsulfonyloxy group is cyclized, the methanesulfonyloxy group for L being particularly preferred. Furthermore it is preferred to cyclize the 1-[amino]-2-[6'-(chloro)-pyrid-3'-yl]-cyclohexane, 4-substituted by a leaving group, of general formula I at an elevated temperature, particularly at the boiling point of the reaction mixture. Advantageously the cyclization is performed in a preferably anhydrous, aprotic solvent. For this purpose particularly halogenated hydrocarbons, most particularly methylene chloride, chloroform or chlorobenzene, or aromatic hydrocarbons, most particularly benzene, toluene or xylene, or a mixture thereof are used. Above all the cyclization is carried out in benzene, toluene and/or xylene as aprotic solvent(s). The cyclization may be carried out under an inert gas atmosphere, e. g. under argon. The epi-epibatidine of formula XIII thus obtained can be isolated from the reaction mixture by cooling the latter to room temperature, shaking it with an aqueous alkali hydroxide solution, separating the phases, extracting the aqueous phase with an appropriate organic solvent, for example, dichloromethane, and washing, drying and evaporating the combined organic phases. The thus obtained epi-epibatidine of formula XIII is optionally purified by crystallization or column chromatography.

The optically active forms of epibatidine of formula XIV may be prepared by several methods.

According to variant b) of the process according to the invention 1-[6'-(chloro)-pyrid-3'-yl)-3-[oxo]-6-[nitro]-hexa-1-ene of formula V is subjected to cyclisation in the presence of an optically active base to yield an optically active 1-[nitro]-2-[6'-(chloro)-pyrid-3'-yl]-cyclohexane-4-one of formula IV. As optically active base preferably (+)-α-(phenyl)-ethyl-amine or (-)-α-(phenyl)-ethyl-amine is used. The reaction may be carried out in the presence of an inert organic solvent, preferably an ether, e. g. tetrahydrofurane or dioxane, or a mixture of such.

In variant b), part α) of the process according to the invention the selective reduction of the 1-[nitro]-2-[6'-(chloro)-pyrid-3'-yl]-cyclohexane-4-one of formula IV to the 1-[nitro]-2-[6'-(chloro)-pyrid-3'-yl]-cyclohexane-4-ol of formula III is preferably carried out with complex metal hydrides. As reducing agent particularly sodium borohydride or lithium, sodium and potassium tri-2-butyl borohydride [L-selectride®] is applied. The reduction with sodium borohydride proved to be most particularly preferable. When sodium borohydride is applied as reducing agent the reduction is preferably performed in a C₁₋₄-alcohol, particularly ethanol. Advantageously one proceeds under cooling, preferably at a temperature of about 0° C. When the reaction has been accomplished the excess of the reducing agent may be decomposed by the addition of a solvent containing an oxo group, e. g. acetone, the solvent removed and the hydroxy compound 1-[nitro]-2-[6'-(chloro)-pyrid-3'-yl]-cyclohexane-4-ol of formula III isolated by dissolving the evaporation residue in a solvent non-miscible with water, e. g. a halogenated hydrocarbon, particularly chloroform, aromatic hydrocarbon, ether, or ethyl acetate, or a mixture of such and washing, drying and evaporating the organic phase. The 1-[nitro]-2-[6'-(chloro)-pyrid-3'-yl]-cyclohexane-4-ol of formula III thus obtained is optionally purified by crystallization or chromatography.

Preferably in variant b), part β) of the process according to the invention the introduction of the leaving group into the optically active nitroalcohol 1-[nitro]-2-[6'-(chloro)-pyrid-3'-yl]-cyclohexane-4-ol of formula III thus obtained to yield a 1-[nitro]-2-[6'-(chloro)-pyrid-3'-yl]-cyclohexane, 4-substituted by a leaving group, of general formula II is carried out with the corresponding sulfonyl halide, particularly methanesulfonyl chloride. The acylating agent is preferably applied in excess. The reaction can be carried out in an apolar aprotic solvent, e. g. halogenated hydrocarbon, such as dichloromethane or chloroform, or a mixture of such in the presence of a base, e. g. pyridine. It is preferable to perform the reaction in a mixture of dichloromethane and pyridine. The reaction can be carried out at a temperature of about room temperature. The 1-[nitro]-2-[6'-(chloro)-pyrid-3'-yl]-cyclohexane, 4-substituted by a leaving group, of general formula II thus obtained can be isolated from the reaction mixture by removing the solvent, dissolving the residue in an organic solvent non-miscible with water, e. g. halogenated hydrocarbon, particularly chloroform, aromatic hydrocarbon, ether or ethyl acetate, or a mixture of such, extracting the solution with an inorganic base, e. g. alkali carbonate, extracting the aqueous solution with the organic solvent non-miscible with water which has been previously used and washing, drying and evaporating the combined organic phases. The thus-obtained 1-[nitro]-2-[6'-(chloro)-pyrid-3'-yl]-cyclohexane, 4-substituted by a leaving group, of general formula II can optionally be purified by crystallization or column chromatography.

Advantageously in variant b), part γ) of the process according to the invention the reduction of the optically active 1-[nitro]-2-[6'-(chloro)-pyrid-3'-yl]-cyclohexane, 4-substituted by a leaving group, of general formula II to the optically active 1-[amino]-2-[6'-(chloro)-pyrid-3'-yl)-cyclohexane, 4-substituted by a leaving group, of formula I is carried out by catalytic hydrogenation or reduction with chemical agent. When a chemical reducing agent is used, preferably the reduction of the nitro group of the optically active 1-[nitro]-2-[6'-(chloro)-pyrid-3'-yl]-cyclohexane, 4-substituted by a leaving group, of formula II is carried out by Bechamps reduction, or with zinc in glacial acetic acid or with zinc, iron or tin in hydrochloric acid or with stannous(II)-chloride the latter being particularly preferred. One may work preferably by using stannous(II)-chloride in a polar organic solvent, e. g. a C₁₋₄-alkanol or tetrahydrofurane, or a mixture of such. Chemical reduction may be performed in a neutral medium as well. The reduction having been completed the reaction mixture may be evaporated.

In variant b), part δ) of the process according to the invention advantageously the cyclization of the optically active 1-[amino]-2-[6'-(chloro)-pyrid-3'-yl]-cyclohexane, 4-substituted by a leaving group, of formula I to the optically active epi-epibatidine of formula XIII is carried out according to the preferences for variant a), part α) of the process according to the invention.

Optically active epibatidine of formula XIV can be prepared by separating racemic epibatidine of formula XIV into the optically active isomers or subjecting a racemic intermediate at a suitable stage of the synthesis to resolution and converting the optically active intermediate thus obtained into optically active epibatidine of formula XIV.

Racemic epi-epibatidine of formula XIII may be subjected to resolution. The process may be carried out by reacting racemic epi-epibatidine of formula XIII with an optically active acid, e. g. optically active tartaric acid, dibenzoyl-tartaric acid or di-0,0'-(p-toluyl)-tartaric acid, separating the diastereoisomeric salts thus formed, e. g. by fractionated crystallization, and liberating the optically active epi-epibatidine of formula XIII from the salt thereof. Then one may proceed to epimerisation of the latter according to variant b)ε) of the process according to the invention.

According to a further embodiment of the process according to the invention another intermediate of chiral structure is subjected to resolution and the optically active intermediate thus obtained is converted into the desired optically active end-product optically active epibatidine of formula XIV in an analogous manner to the process described above for the preparation of racemic epibatidine of formula XIV.

Thus according to a preferred embodiment of the invention optically active epibatidine of formula XIV may be prepared by carrying out the variant b), parts α) to ε) of the process according to the invention with the optically active 1-[nitro]-2-[6'-(chloro)-pyrid-3'-yl]-cyclohexane-4-one of formula IV as above described.

Moreover one may proceed preferably by subjecting the racemic nitro alcohol 1-[nitro]-2-[6'-(chloro)-pyrid-3'-yl]-cyclohexane-4-ol of formula III to resolution and carrying out the further steps of the synthesis by using the optically active 1-[nitro]-2-[6'-(chloro)-pyrid-3'-yl]-cyclohexane-4-ol of formula III thus obtained. The racemate of 1-[nitro]-2-[6'-(chloro)-pyrid-3'-yl]-cyclohexane-4-ol of formula III is separated into the optically active isomers preferably by acylating the racemic 1-[nitro]-2-[6'-(chloro)-pyrid-3'-yl]-cyclohexane-4-ol of formula III with optically active menthyl-chloro-formiate (chloro-formic acid menthyl ester), separating the diastereomers thus formed by crystallization and thereafter removing the menthyl group to yield the desired optically active 1-[nitro]-2-[6'-(chloro)-pyrid-3'-yl]-cyclohexane-4-ol of formula III. The further reactions may be according to variant b), parts β), γ), δ) and ε) of the process according to the invention indicated above with the preferences described with regard to these.

The 1-[amino]-2-[6'-(chloro)-pyrid-3'-yl]-cyclohexanes, 4-substituted by a leaving group, of general formula I used as starting substances in variant a) of the process according to the invention could have been prepared by selectively reducing the nitro group of 1-[nitro]-2-[6'-(chloro)-pyrid-3'-yl]-cyclohexane, 4-substituted by a leaving group, of general formula II. Advantageously this reaction is performed by catalytic hydrogenation or reduction with a chemical agent. Preferably this selective reduction is carried out by Bechamps-reduction, or with zinc in glacial acetic acid or with zinc, iron or tin in hydrochloric acid or with stannous(II)-chloride the latter being particularly preferred. The reduction with stannous(II)-chloride is preferably carried out in a polar organic solvent, e. g. C₁₋₄-alcohol, particularly ethanol, or tetrahydrofurane, or a mixture of such. The chemical reduction can also be carried out in a neutral reaction medium. The reduction reaction with stannous(II)-chloride can be carried out under heating, preferably at the boiling point of the reaction mixture. The thus obtained 1-[amino]-2-[6'-(chloro)-pyrid-3'-yl]-cyclohexane, 4-substituted by a leaving group, of general formula I can be isolated from the reaction mixture by cooling the mixture to room temperature and adding a solvent non-miscible with water, e. g. a chlorinated hydrocarbon, preferably chloroform, or a mixture of such to it and rendering the solution slightly alkaline. The separated precipitate is filtered off, washed with a solvent non-miscible with water and the organic phases are combined, washed, dried and evaporated. The thus-obtained 1-[amino]-2-[6'-(chloro)-pyrid-3'-yl]-cyclohexane, 4-substituted by a leaving group, of general formula I is optionally purified by crystallization or column chromatography.

The starting substances of 1-[nitro]-2-[6'-(chloro)-pyrid-3'-yl]-cyclohexanes, 4-substituted by a leaving group, of general formula II for preparing the above starting substances could be prepared as specified below.

A particular advantage of the synthesis resides in the fact that 1-(nitro)-pentane-4-one of formula 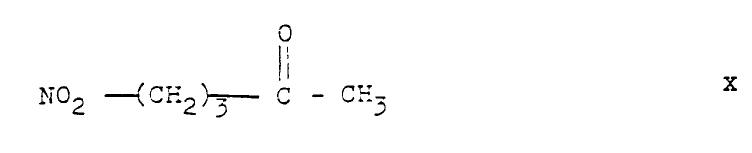 is used as starting substance, which can be obtained by reacting commercially readily available compounds, that is nitromethane of formula

H₃C - NO₂ XII

and methylvinylketone of formula 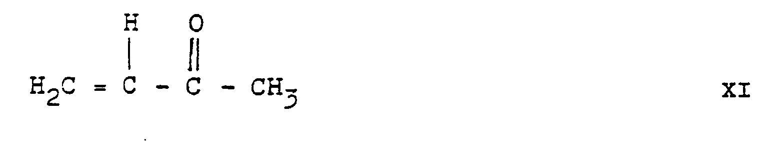 The 1-(nitro)-pentan-4-one of formula X can be produced according to the method of D. E. Bergbreiter and J. J. Lalonde (J. Org. Chem. 52 [1987], 1 601 to 1 603).

In the next reaction step the 1-(nitro)-pentane-4-one of formula X is brominated. Preferably the bromination is carried out with elementary bromine in a lower alcohol, particularly methanol. Conveniently the bromination is performed at a temperature of about room temperature taking care of that the temperature of the reaction mixture should not rise above 40°C. The acetalic ether bond being formed in the reaction is hydrolyzed. The 1-(bromo)-5-(nitro)-pentane-2-one of formula 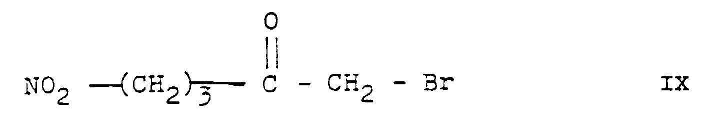 thus obtained may be isolated by extracting the aqueous solution with a solvent non-miscible with water such as chlorinated hydrocarbon, aromatic hydrocarbon, ethyl acetate or preferably ether, washing the extract first acid-free with a caustic solution then neutral with water, dried and evaporated. The 1-(bromo)-5-(nitro)-pentane-2-one of general formula IX is optionally purified by column chromatography.

The 1-(bromo)-5-(nitro)-pentane-2-one of formula IX is then reacted with a triaryl phosphine to obtain a phosphonium salt [5- (nitro) -pentane-2-one]-triaryl-phosphonium bromide of general formula 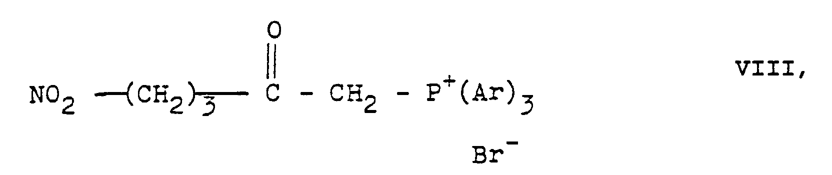 wherein
- Ar: stands for an aryl group, preferably phenyl group.
The reaction is preferably carried out with triphenyl phosphine. It is advantageously performed in an apolar aprotic solvent, preferably an aromatic hydrocarbon, particularly benzene or a mixture of such. It is preferable to carry out the reaction by adding to the solution of the 1-(bromo)-5-(nitro)-pentane-2-one of formula IX in an apolar aprotic solvent a solution of triphenyl phosphine in the same solvent. The reaction may be carried out at a temperature of from 10 to 30°C, preferably at room temperature. The oily product gets crystalline upon standing. The crystalline [5-(nitro)-pentane-2-one]-triaryl-phosphonium bromide of general formula VIII can be filtered off and washed.

The phosphonioum salt [5-(nitro)-pentane-2-one]-triaryl-phosphonium bromide of general formula VIII is then converted into a [5-(nitro)-pentane-2-one]-triaryl-phosphorane, preferably [5-(nitro)-pentane-2-one]-triphenyl-phosphorane, of general formula 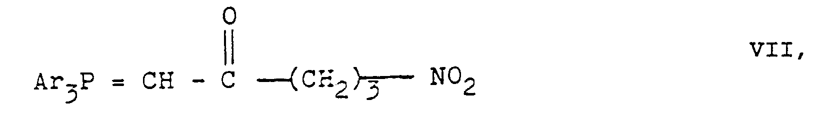 wherein
- Ar: stands for an aryl group, preferably phenyl group.
The phosphonium salt [5-(nitro)-pentane-2-one]-triaryl-phosphonium bromide is dissolved in an apolar aprotic solvent non-miscible with water, preferably in a halogenated aliphatic hydrocarbon, such as dichloromethane, or a mixture of such and stirred with a diluted alkali hydroxide solution, e. g. sodium or potassium hydroxide solution, at a temperature of about room temperature. The phases are then separated, the organic layer is washed, dried and evaporated.

The [5- (nitro) -pentane-2-one]-triaryl-phosphorane, preferably [5-(nitro)-pentane-2-one]-triphenyl-phosphorane, of general formula VII thus obtained is reacted with 6-(chloro)-pyridine-3-aldehyde [6-(chloro)-nicotinic aldehyde] of formula 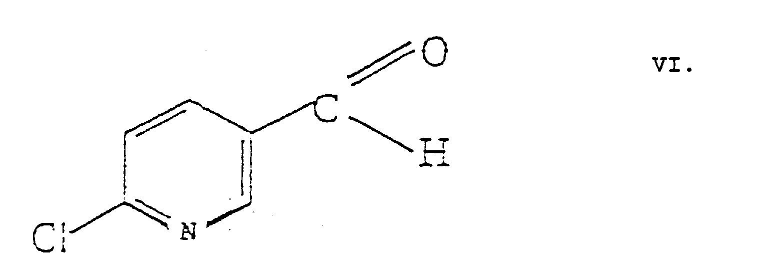 The reaction can be performed in an anhydrous aprotic solvent, preferably a halogenated aliphatic hydrocarbon, such as dichloromethane, or a mixture of such. The reaction can be carried out under heating, preferably at the boiling point of the reaction mixture. It is preferable to perform the reaction by adding to the solution of the [5-(nitro)-pentane-2-one]-triaryl-phosphorane, preferably [5-(nitro)-pentane-2-one]-triphenyl-phosphorane, of general formula VII in an anhydrous aprotic solvent the solution of the 6-(chloro)-pyridine-3-aldehyde of formula VI in the same solvent. The reaction mixture is then preferably worked up by cooling, washing and evaporating. The olefine 1-[6'-(chloro)-pyrid-3'-yl]-3-(oxo)-6-(nitro)-hexa-1-ene of formula 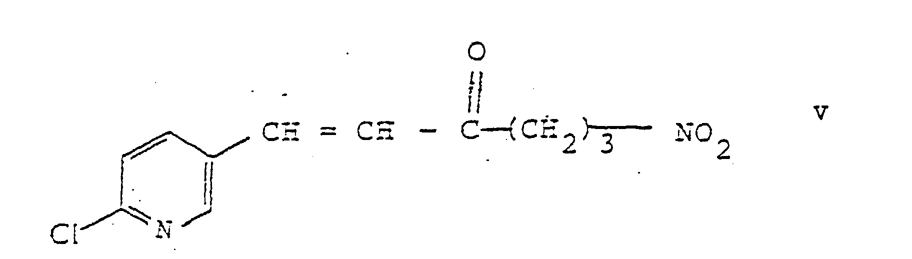 thus obtained can be purified by crystallization or column chromatography.

The 6-(chloro)-pyridine-3-aldehyde of formula VI could be prepared readily from the commercially available 6-(chloro)-nicotinic acid by methods known from the literature (F. E. Ziegler, J. G. Sweeny: Tetrahedron Letters, [1969], 1 097 to 1 110).

The olefine 1-[6'-(chloro)-pyrid-3'-yl]-3-(oxo)-6-(nitro)-hexa-1-ene of formula V obtained as specified above is then subjected to cyclization. This reaction may be carried out in an anhydrous aprotic organic solvent. As reaction medium preferably cyclic ethers, e. g. tetrahydrofuran, or a mixture of such can be applied. The cyclization is preferably carried out in the presence of a base, particularly potassium fluoride applied onto a basic aluminum oxide carrier (D. E. Bergbreiter, J. J. Lalonde: J. Org. Chem. 52 [1987], 1 601 to 1 603). The cyclization may be carried out at a temperature of about room temperature. The thus-obtained nitroketone 1-[nitro]-2-[6'-(chloro)-pyrid-3'-yl]-cyclohexane-4-one of formula IV may be isolated by removing the base and evaporating the solution. Optionally the 1-[nitro]-2-[6'-(chloro)-pyrid-3'-yl]-cyclohexane-4-one of formula IV thus obtained can be purified by crystallization or column chromatography.

The further reactions are as those of variant b), parts α), β) and γ) of the process according to the invention with the preferences indicated there also including the racemic substances.

As results therefrom simultaneously part of this synthesis of the starting substances 1-[amino]-2-[6'-(chloro)-pyrid-3'-yl]-cyclohexanes, 4-substituted by a leaving group, of formula I for variant a) of the process according to the invention is for the synthesis of the starting substance 1-[6'-(chloro)-pyrid-3'-yl]-3-[oxo]-6-[nitro]-hexa-1-ene of formula V.

The invention is illustrated in detail with the aid of the following Examples. In these the ratios of the components of the mixtures for chromatography are always by volume. Moreover in the following Examples unless other indications have been made column chromatography is to be meant as having been carried out on silica gel with the designation "Kieselgel 60" (0.063 to 0.200 mm). Furthermore "brine" is a saturated sodium chloride solution. For the potassium fluoride precipitated on aluminum oxide always the total amount of these 2 substances and the potassium fluoride contents thereof have been indicated.

In a manner known per se the free epibatidine of formula XIV can be converted into an acid addition salt, particularly hydrochloride salt, and/or the free epibatidine of formula XIV can be liberated from an acid addition salt of it and/or converted into another acid addition salt thereof.

### Example 1

### (±)-Epibatidine

### Step 1

### (±)-Epi-epibatidine

1.1 g (0.0036 mole) of (±)-1α-[amino]-2β-[6'-(chloro)-pyrid-3'-yl]-4β-[methanesulfonyloxy]-cyclohexane prepared as indicated below are dissolved in 150 ml of anhydrous toluene and the reaction mixture is heated to boiling under argon overnight. The reaction mixture is then cooled, 25 ml of a 5% by weight sodium hydroxyde solution are added, the phases are thoroughly shaken, the layers are separated. The aqueous phase is extracted ten times with 20 ml of dichloromethane each. The combined organic layers are washed twice with 100 ml of water each and 100 ml of a saturated sodium chloride solution, dried over magnesium sulfate and evaporated. The residue is subjected to chromatography on a silica column and eluted with a 1:1 mixture of chloroform and methanol. Thus 350 mg of the desired compound are obtained, yield 46%, faint yellow oil. R_{f} = 0.35 (1:1 mixture of chloroform and methanol).
IR_{(film)}: 3260, 3220, 1580, 1560, 1760, 1200, 1100 cm⁻¹.

The (±)-1α-[amino]-2β-[6'-(chloro)-pyrid-3'-yl]-4β-[methanesulfonyloxy]-cyclohexane used as starting substance has been prepared as follows.

### a) 1-(Bromo)-5-(nitro)-pentane-2-one

80.0 g (0.61 mole) of 1-(nitro)-pentane-4-one are dissolved in 250 ml of anhydrous methanol, whereupon 31.5 ml (0.61 mole) of bromine are quickly added under cooling with ice. The reaction mixture is stirred for a further 2 hours at a rate that the internal temperature should not exceed 40°C. To the reaction mixture 250 ml of water are added, the reaction mixture is stirred at room temperature overnight. Next morning the solution is extracted three times with 300 ml of ether each, the ethereal solution is washed with a 10% by weight aqueous sodium carbonate solution free of acid, whereupon it is washed three times with 200 ml of water each and 200 ml of a saturated sodium chloride solution, dried over calcium chloride and evaporated. The dry residue is subjected to chromatography on a silica column and eluted with a 3:1 mixture of n-hexane and ethyl acetate. Thus 70.4 g of the desired compound are obtained in the form of a faint yellow liquid, yield 55%. R_{f} = 0.30.
IR_{(film)}: 2950, 1720, 640 cm⁻¹.

### b) [5-(Nitro)-pentane-2-one]-triphenyl-phosphonium bromide

10.25 g (0.048 mole) of the bromine compound 1-(bromo)-5-(nitro)-pentane-2-one prepared according to section a) are dissolved in 30 ml of anhydrous benzene whereupon a solution of 14.09 g (0.0537 mole) of triphenyl phosphine in 50 ml of anhydrous benzene is added dropwise. The reaction mixture is stirred at room temperature for 48 hours whereby the oily precipitate becomes crystalline. The precipitated salt is filtered and washed with n-hexane. Thus 20.5 g of the desired compound are obtained,
yield 89%, mp.: 70-72°C.

### c) [5-(Nitro)-pentane-2-one]-triphenyl-phosphorane

8.1 g (0.0171 mole) of the phosphonium salt [5-(nitro)-pentane-2-one]-triphenyl-phosphonium bromide prepared according to section b) are dissolved in 160 ml of dichloromethane and the solution formed is stirred with 136 ml (0.0542 mole) of a 1% by weight aqueous sodium hydroxide solution for 30 minutes. The two phases are separated, the dichloromethane layer is washed three times with 100 ml of water each and with 100 ml of a saturated sodium chloride solution, dried over calcium chloride and evaporated. The dry residue is thoroughly triturated with n-hexane. Thus 4.8 g of the desired compound are obtained, yield 72%, mp.: 94-97°C.

### d) 1-[6'-(Chloro)-pyrid-3'-yl]-3-[oxo]-6-[nitro]-hexa-1-ene

To a solution of the 13.5 g (0.0344 mole) of the [5-(nitro)-pentane-2-one]-triphenyl-phosphorane prepared according to section c) in 70 ml of anhydrous dichloromethane a solution of 3.1 g (0.022 mole) of 6-(chloro)-pyridine-3-aldehyde in 70 ml of anhydrous dichloromethane is added. The reaction mixture is heated to boiling for 8 hours in argon atmosphere. The reaction mixture is cooled, the dichloromethane solution is washed subsequently three times with 150 ml of water each and 150 ml of a saturated sodium chloride solution, dried over calcium chloride and evaporated. The dry residue is subjected to chromatography on a silica column and eluted with a 1:1 mixture of n-hexane and ethyl acetate. Thus 4.7 g of the pure desired compound are obtained, yield 84%. Mp.: 97-100°C. R_{f} = 0.52.
IR_{(KBr)}: 1700, 1680, 1620, 1580, 1550, 1100 cm⁻¹.

### e) (±)-1α-[Nitro]-2β-[6'-(chloro)-pyrid-3'-yl]-cyclohexane-4-one

1.6 g (0.0063 mole) of 1-[6'-(chloro)-pyrid-3'-yl]-3-[oxo]-6-[nitro]-hexa-1-ene prepared according to section d) are dissolved in 50 ml of anhydrous tetrahydrofurane whereupon 4.0 g (0.089 mole) of potassium fluoride precipitated on aluminum oxide containing 0.0117 mole of potassium fluoride are added. The reaction mixture is stirred at room temperature overnight. The solid product is filtered, washed with 300 ml of ethyl acetate. The combined filtrates are dried over calcium chloride and evaporated. The residue is purified by chromatography on a silica column and elution with a 1:1 mixture of n-hexane and ethyl acetate. Thus 1.1 g of the pure desired product are obtained, yield 59%.
Mp.: 118-121°C.
R_{f} = 0.38. IR_{(KBr)}: 1710, 1585, 1550, 1100 cm⁻¹.

### f) (±)-1α-[Nitro]-2β-[6'-(chloro)-pyrid-3'-yl]-cyclohexane-4β-ol

2.8 g (0.0110 mole) of (±)-1α-[nitro]-2β-[6'-(chloro)-pyrid-3'-yl]-cyclohexane-4-one prepared according to section e) are dissolved in 200 ml of anhydrous ethanol whereupon 1.2 g (0.0317 mole) of sodium borohydride are added within a period of about one hour and a half in small portions. The excess of the reducing agent is decomposed by careful addition of acetone, the reaction mixture is evaporated in vacuo, the solid residue is dissolved in a mixture of 50 ml of water and 200 ml of chloroform, the mixture is thoroughly shaken and the layers are separated. The aqueous phase is extracted three times with 200 ml of chloroform each. The combined organic layers are washed twice with 200 ml of water each and 100 ml of a saturated sodium chloride solution, dried over calcium chloride and evaporated. Thus 1.9 g of the desired compound are obtained, yield 67%, mp.: 149-153°C.
IR_{(film)}: 3380, 1580, 1570, 1550, 1100, 1080 cm⁻¹.
R_{f} = 0.42 (10 : 1 mixture of chloroform and methanol).

### g) (±)-1α-[Nitro]-2β-[6'-(chloro)-pyrid-3'-yl]-4β-[methanesulfonyloxy]-cyclohexane

1.0 g (0.003896 mole) of (±)-1α-[nitro]-2β-[6'-(chloro)-pyrid-3'-yl]-cyclohexane-4β-ol prepared according to section f) are dissolved in a mixture of 15 ml of anhydrous dichloromethane and 30 ml of pyridine, whereupon 0.75 ml (0.0097 mole) of methanesulfonyl chloride is added dropwise under cooling with icecold water. The reaction mixture is stirred at room temperature overnight and thereafter the solvent is removed in vacuo. The dry residue is dissolved in a mixture of 50 ml of chloroform and 25 ml of a 10% by weight sodium carbonate solution, the mixture is thoroughly shaken, the phases are separated. The aqueous phase is extracted three times with 50 ml of chloroform each. The combined organic layers are washed three times with 100 ml of water each and 100 ml of a saturated sodium chloride solution, dried over calcium chloride and evaporated. The dry residue is subjected to chromatography on a silica column and eluted with a 1:1 mixture of n-hexane and ethyl acetate. Thus 1.18 g of the desired compound are obtained, yield 91%, mp.: 120-122°C.
R_{f} = 0.46
IR_{(KBr)}: 1590, 1570, 1540, 1530, 1450, 1350, 1180, 1090 cm⁻¹.

### h) (±)-1α-[Amino]-2β-[6'-(chloro)-pyrid-3'-yl]-4β-[methanesulfonyloxy]-cyclohexane

1.5 g (0.0048 mole) of (±)-1α-[nitro]-2β-[6'-(chloro)-pyrid-3'-yl]-4β-[methanesulfonyloxy]-cyclohexane prepared according to section g) are dissolved in 150 ml of ethanol whereupon 10.76 g (0.0477 mole) of stannous(II)-chloride dihydrate are added. The reaction mixture is heated to boiling for 24 hours, whereupon it is cooled, 200 ml of chloroform are added and the pH is adjusted to 9 by adding a concentrated ammonium hydroxide solution. The precipitated product is filtered, washed with 400 ml of chloroform, the organic phase is washed twice with 200 ml of water and once with 200 ml of a saturated sodium chloride solution, dried over magnesium sulfate and evaporated. Thus 1.1 g of the desired compound are obtained in the form of a colourless oil, yield 80%.
R_{f} = 0.69 (chloroform : methanol = 10 : 1).

### Step 2

### (±)-Epibatidine

20 mg (0.09 mmole) of epi-epibatidine prepared according to step 1 are boiled in 3 ml of anhydrous tert.butanol in the presence of 100 mg of potassium tert.butylate for 30 hours. After evaporation the product is purified by chromatography on a silica gel column (eluent: 1:1 mixture of benzene and methanol). Yield: 10 mg (50%), R_{f} = 0.25.

### Example 2

### Step 1

### (-)-1α-[Nitro]-2β-[6'-(chloro)-pyrid-3'-yl]-cyclohexane-4-one

5.0 g (0.01963 mole) of 1-[6'-(chloro)-pyrid-3'-yl]-3-[oxo]-6-[nitro]-hexa-1-ene prepared as described in the preparation of the starting substance of Example 1, sections a) to d) are dissolved in 50 ml of anhydrous tetrahydrofurane, whereupon 12.6 ml (0.09815 mole) of (+)-α-(phenyl)-ethyl-amine are added. The reaction mixture is allowed to stand at room temperature for 3 days, whereupon the solution is evaporated. The product is purified by subjecting the crude product to chromatographic on a silica column and eluting first with a 10:1 mixture of benzene and methanol (R_{f} = 0.38) and thereafter with a 1:1 mixture of n-hexane and ethyl acetate (R_{f} = 0.42). The product is crystallized from ethanol. Thus 1.45 g of the pure desired compound are obtained, yield 29%, mp.: 149-151°C, (α)$\frac{\text{V}}{\text{0}}$ = -86.2° (c=2, chloroform).

Further steps according to variant b), parts α) to ε) result in epibatidine.

### Example 3

### Step 1

### (-) -1α-[Nitro]-2β-[6'-(chloro)-pyrid-3'-yl]-cyclohexane-4β-yl-carbonic acid menthyl ester

1.536 g (6 millimoles) of racemic (±)-1α-[nitro]-2β-[6'-(chloro)-pyrid-3'-yl]-cyclohexane-4β-ol prepared as described in the preparation of the starting substance of Example 1, sections a) to f) are dissolved in a mixture of 30 ml of anhydrous dichloromethane and 1.4 ml of pyridine at room temperature. To the solution 3.0 ml (14 millimoles) of (-)-menthyl-chloro-formiate (Aldrich 24,530-5) are added dropwise. The reaction mixture is stirred at room temperature for 6 hours, whereupon further 0.2 ml of the reactant is added. The reaction mixture is allowed to stand overnight and then evaporated to dryness in vacuo. The residue is dissolved in a mixture of 60 ml of chloroform and 5 ml of water. The pH of the aqueous layer is adjusted to 9 with a 5% by weight sodium hydrogen carbonate solution. The layers are separated, the chloroform solution is washed three times with 20 ml of water each, dried over sodium sulfate, filtered and evaporated in vacuo. The residue is crystallized from 100 ml of methanol under clarification with charcoal. Thus 1.2 g of the desired compound are obtained in the form of white crystals, yield 45.8%, mp.: 98-100°C, [α]_{D}²⁵ = -56.0° (c = 0.5, chloroform).

The product thus obtained is further purified by column chromatography (Merck 9385 silicagel, water pump vacuo; eluent = benzene : ethyl acetate = 19:1). The fractions containing the desired compound are evaporated. Thus 630 mg of an oil are obtained which is crystallized from methanol. Thus 280 mg of the desired compound are obtained, mp.: 183-184°C, [α]_{D}²⁵ = -36.7° (c = 0.5 chloroform).

### Step 2

### (+)-1α-[Nitro]-2β-[6'-(chloro)-pyrid-3'-yl]-cyclohexane-4β-ol

100 mg of the crystalline substance (-)-1α-[nitro]-2β-[6'-(chloro)-pyrid-3'-yl]-cyclohexane-4β-yl-carbonic acid menthyl ester {[α]_{D}²⁵ = -36.7 (c = 0.5 chloroform)} prepared according to step 1 are dissolved in a mixture of 20 ml of 10% by weight sulfuric acid and 20 ml of ethanol and the solution is heated to boiling for 24 hours, whereupon the ethanol is removed in vacuo. To the aqueous residue about 30 ml of benzene are added and the mixture is evaporated again to dryness in vacuo. This operation is carried out five or six times. The residue is suspended in 30 ml of chloroform and the pH is adjusted to a value of about 10 by adding a concentrated ammonium hydroxide solution. The layers are separated, the chloroform solution is washed with water, dried over sodium sulfate, filtered and evaporated in vacuo. The residue is purified by column chromatography (Merck 9385 silicagel, water pump vacuo; eluent : chloroform : methanol = 20:1). The fractions containing the desired compound are combined and evaporated. The residue is crystallized from ether. Thus 32 mg of the desired compound are obtained, mp.: 190-194°C, [α]_{D}²⁵ = +63.9° (c = 0.5, chloroform).

### Further steps

The (+)-1α-[nitro]-2β-[6'-(chloro)-pyrid-3'-yl]-cyclohexane-4β-ol was converted into the corresponding epibatidine by working analogously to Example 1 starting with reacting the former instead of (±)-1α-[nitro]-2β-[6'-(chloro)-pyrid-3'-yl]-cyclohexane-4β-ol in the description of the preparation of the starting substance of Example 1, section f).

### Example 4

### A) (-)-Epibatidine and (+)-epibatidine dihydrochloride

### Step 1

### (+)-Epi-epibatidine and (+)-epi-epibatidine dihydrochloride

1.00 g (5 millimoles) of racemic epi-epibatidine prepared according to Example 1 are dissolved in 20 ml of hot acetone, whereupon a solution of 0.96 g (2.5 millimoles) of (-)-di-0,0'-p-toluoyl-L-tartaric acid in a mixture of 20 ml of acetone and 10 ml of water is added. The solution remains clear for some minutes but later the precipitation of crystals starts from the hot solution. The mixture is allowed to cool to room temperature and allowed to stand overnight in a refrigerator. Next morning the precipitated crystals are filtered, washed with a mixture of 5 ml of acetone and 1 ml of water and dried. Thus 1.053 g of the salt are obtained. Mp.: 188-190°C, [α]_{D}²⁵ = -56.9° (c = 0.5, methanol).

1.00 g of the above salt is dissolved in a hot mixture of 100 ml of ethanol and 5 ml of water and the solution is allowed to stand at room temperature for a week-end. The precipitated crystals are filtered, washed with a mixture of 5 ml of ethanol and 0.5 ml of water and dried. Thus 341 mg of the salt are obtained, mp.: 200-201°C, [α]_{D}²⁵ = -53.4° (c = 0.5, methanol).

300 mg of the above salt are suspended in a mixture of 120 ml of chloroform and 8 ml of water at room temperature and sufficient amount of a 1 molar sodium hydroxide solution (about 0.2-0.3 ml) is added to adjust the pH of the aqueous phase to 9-10. The layers are separated, the chloroform solution is washed twice with 8 ml of water each, dried over sodium sulfate, filtered and evaporated in vacuo. Thus 155 mg of an oily product are obtained, [α]_{D}²⁵ = +36.1° (c = 0.5, methanol).

### Step 2

### (-)-Epibatidine and (+)-epibatidine dihydrochloride

On epimerizating the (+)-epi-epibatidine prepared according to step 1 analogously to Example 1, step 2 (-)-epibatidine is obtained which is converted into the hydrochloride. The rotation of the salt {[α]_{D}²⁵ = +34.8° (c = 0.36, methanol)} corresponds to the value {[α]_{D}²⁵ = +34.7° (c = 0.36, methanol)} disclosed in the Prior Art (S.R. Fletscher et al., J. Chem. Soc. Chem. Column. [1993], 1216).

### B)

### (+)-Epibatidine

### Step 1

### (-)-Epi-epibatidine

The first crystallization mother-lye obtained by the preparation of (+)-epi-epibatidine under section A) is evaporated to dryness in vacuo and the crystalline residue is made water-free by evaporation on a rotating evaporator several times. Thus 649 mg of the product are obtained, mp.: 164-176°C, [α]_{D}²⁵ = -64.8° (c = 0.5, methanol).

200 mg of the above salt are suspended in a mixture of 80 ml of chloroform and 6 ml of water. The pH is adjusted to 9-10 by adding a 1 molar sodium hydroxide solution. The layers are separated, the chloroform solution is washed with water (about 10 ml), dried over sodium sulfate, filtered and evaporated to dryness. Thus 96 mg of an oily product are obtained, [α]_{D}²⁵ = -17.2° (c = 0.5, methanol).

400 mg of the above salt {[α]_{D}²⁵ = -64.8° (c = 0.5, methanol)} are treated with 2 ml of a 1 M sodium hydroxide solution in a mixture of 150 ml of chloroform and 10 ml of water. The reaction mixture is worked up. 160 mg of an oily product are obtained. [α]_{D}²⁵ = -17.2° (c = 0.5, methanol).

The oily crude product thus obtained (160 mg, 0.76 millimoles) is dissolved in 3.2 ml of hot acetone, whereupon a solution of 153 mg (0.396 millimole) of (+)-di-0,0'-p-toluoyl-D-tartaric acid, 3.2 ml of acetone and 0.6 ml of water is added. The reaction mixture is allowed to cool to room temperature, allowed to stand for a few hours, the precipitated crystals are filtered, washed with some drops of aqueous acetone and dried. Thus 269 mg of the salt are obtained, mp.: 196-198°C, [α]_{D}²⁵ = +63.4° (c = 0.5, methanol).

200 mg of the above salt are recrystallized from a mixture of 4.5 ml of ethanol and 0.5 ml of water. Thus 124 mg of the pure salt are obtained, mp.: 204-205°C, [α]_{D}²⁵ = +60.3°, (c = 0.5, methanol).

100 mg of the salt thus obtained are treated with 2 ml of a 1 M sodium hydroxide solution in a mixture of 40 ml of chloroform and 4 ml of water. Thus 60 mg of an oily product are obtained, [α]_{D}²⁵ = -40.3° (c = 0.5, methanol).

### Step 2

### (+)-Epibatidine

On epimerizating the (-)-epi-epibatidine prepared according to step 1 analogously to Example 1, step 2 (+)-epibatidine is obtained. This could be converted into the (-)-epibatidine hydrochloride.

## Claims

1. Process for preparing racemic or optically active epibatidine of formula 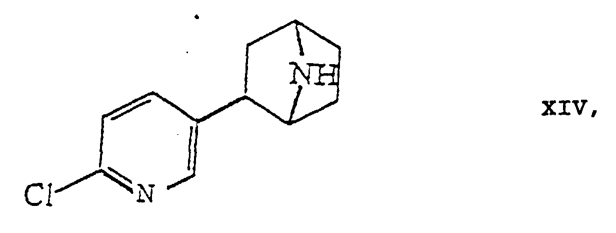 characterized by
a)
α) cyclising a racemic or optically active 1-[amino]-2-[6'-(chloro)-pyrid-3'-yl]-cyclohexane, 4-substituted by a leaving group, of general formula 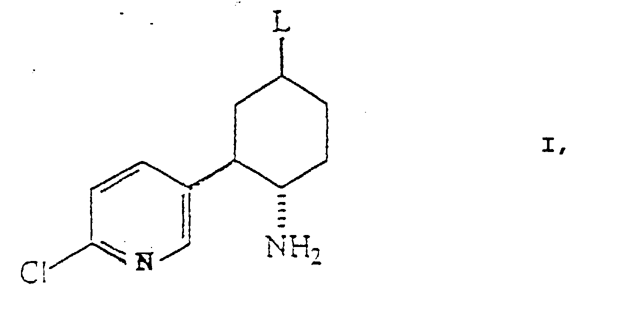 wherein
L stands for a leaving group, particularly a C₁₋₄-alkylsulfonyloxy or arylsulfonyloxy group
to racemic or optically active epi-epibatidine of formula 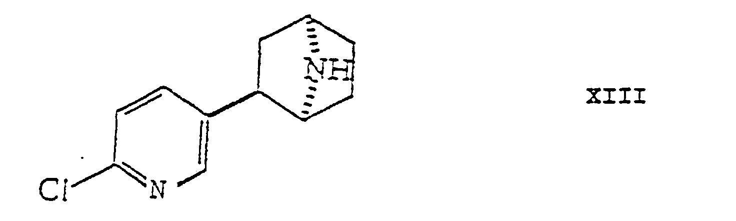 and thereafter
β) subjecting the latter to epimerization in the presence of a base
or
b) cyclizing 1-[6'-(chloro)-pyrid-3'-yl]-3-[oxo]-6-[nitro]-hexa-1-ene of formula 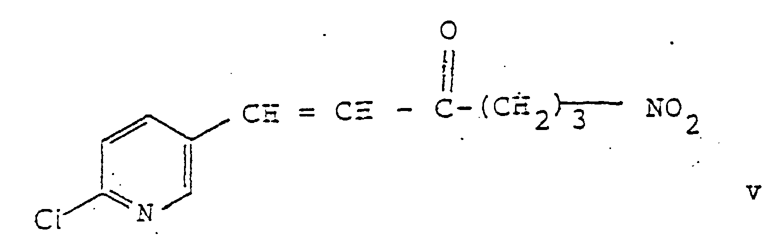 in the presence of an optically active base to optically active 1-[nitro]-2-[6'-(chloro)-pyrid-3'-yl]-cyclohexane-4-one of formula 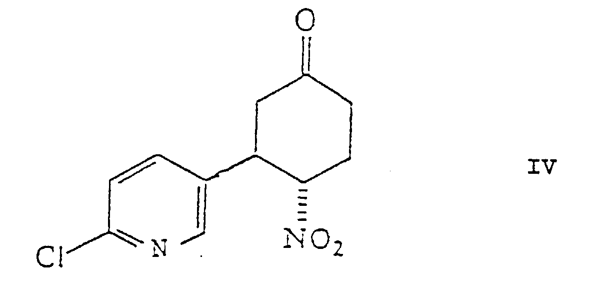 and thereafter
α) selectively reducing the optically active 1-[nitro]-2-[6'-(chloro)-pyrid-3'-yl]-cyclohexane-4-one of formula IV thus obtained to yield optically active 1-[nitro]-2-[6'-(chloro)-pyrid-3'-yl]-cyclohexane-4-ol of formula 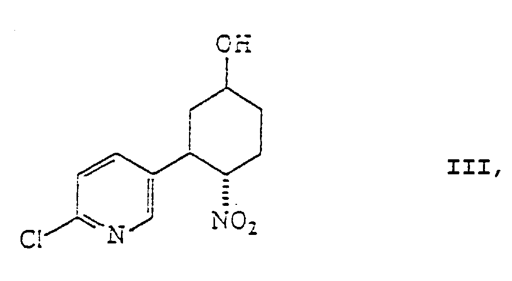
β) introducing into the latter a leaving group to yield an optically active 1-[nitro]-2-[6'-(chloro)-pyrid-3'-yl]-cyclohexane, 4-substituted by a leaving group, of general formula 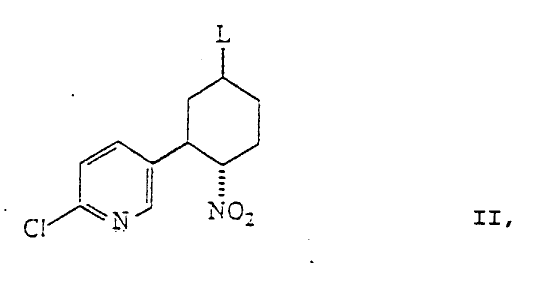 wherein
L represents a leaving group, particularly a C₁₋₄-alkylsulfonyloxy or arylsulfonyloxy group,
γ) reducing the latter to an optically active 1-[amino]-2-[6'-(chloro)-pyrid-3'-yl]-cyclohexane, 4-substituted by a leaving group, of general formula 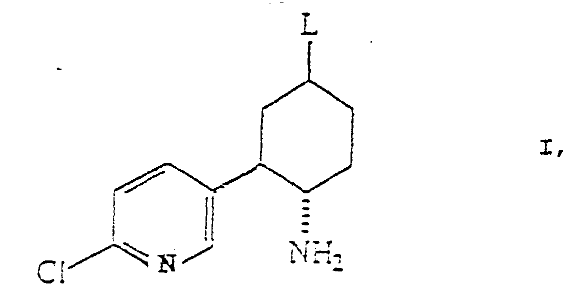
δ) cyclising the latter to an optically active epi-epibatidine of formula XIII
and
ε) subjecting the latter to epimerization in the presence of a base.

2. Process according to claim 1a)β), characterized by carrying out epimerization at an elevated temperature.

3. Process according to claim 1a)β) or 2, characterized by using for the epimerization an alkali alcoholate or another organic alkali compound.

4. Process according to claim 1a)β), 2 or 3, characterized by using for the epimerization as base potassium tertiary butylate, sodium ethylate or lithium diisopropyl amine.

5. Process according to claims 1a)β) or 2 to 4, characterized by carrying out the epimerization in the presence of an alkali alcoholate under heating and the epimerization in the presence of another organic alkali compound at a temperature of about 0°C.

6. Process according to claims 1a)β) or 2 to 5, characterized by carrying out the epimerization in a C₁₋₄-alkanol as medium in the presence of an alkali alcoholate corresponding to the alkanol used as solvent.

7. Process according to claim la), characterized by cyclising a 1-[amino]-2-[6'-(chloro)-pyrid-3'-yl]-cyclohexane, 4-substituted by a leaving group, of general formula I, in which the leaving group represented by L is a methanesulfonyloxy, p-toluenesulfonyloxy or p-(bromo)-phenylsulfonyloxy group.

8. Process according to claim 1a) or 7, characterized by cyclising the 1-[amino]-2-[6'-(chloro)-pyrid-3'-yl]-cyclohexane, 4-substituted by a leaving group, of general formula I at an elevated temperature, particularly at the boiling point of the reaction mixture.

9. Process according to claim 1a), 7 or 8, characterized by carrying out the cyclization in an aprotic solvent, particularly in a halogenated hydrocarbon or an aromatic hydrocarbon or a mixture thereof.

10. Process according to claims 1a) or 7 to 9, characterized by carrying out the cyclization in benzene, toluene and/or xylene as aprotic solvent(s).

11. Process according to claim 1b), characterized by using for the cyclization (+)-α-(phenyl)-ethyl-amine or (-)-α-(phenyl)-ethyl-amine as optically active base.

12. Process according to claim 1, characterized by resolving the racemic 1-[nitro]-2-[6'-(chloro)-pyrid-3'-yl]-cyclohexane-4-ol of formula III and thereafter converting the optically active 1-[nitro]-2-[6'-(chloro)-pyrid-3'-yl]-cyclohexane-4-ol of formula III into optically active epibatidine of formula XIV.

13. Process according to claim 1 or 12, characterized by carrying out the resolving of the racemic 1-[nitro]-2-[6'-(chloro)-pyrid-3'-yl]-cyclohexane-4-ol of formula III by esterifying it with optically active menthyl chloro formiate, separating the diastereomers thus obtained by crystallization and thereafter removing the menthyl group.

## Patentansprüche

1. Verfahren zur Herstellung von racemischem oder optisch aktivem Epibatidin der Formel 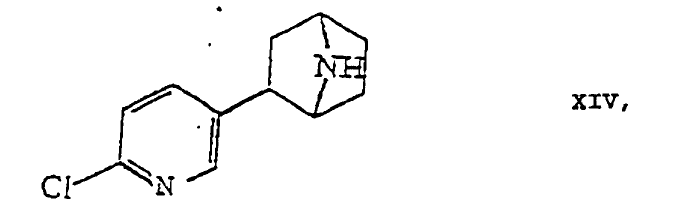 gekennzeichnet durch
a)
α) Cyclisieren eines racemischen oder optisch aktiven 1-[Amino]-2-[6'-(chlor)-pyrid-3'-yl]-cyclohexans, 4-substituiert durch eine Abgangsgruppe, der allgemeinen Formel 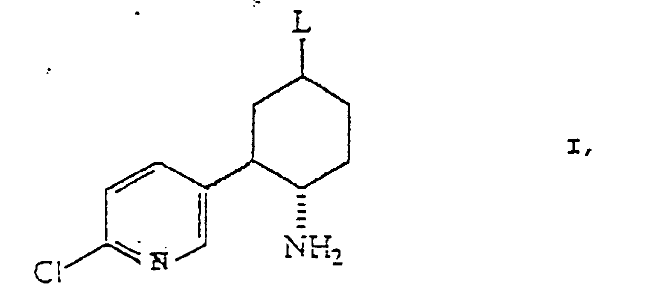 worin
L für eine Abgangsgruppe, insbesondere eine C₁₋₄-Alkylsulfonyloxy- oder Arylsulfonyl oxygruppe steht,
zu racemischem oder optisch aktivem epi-Epibatidin der Formel 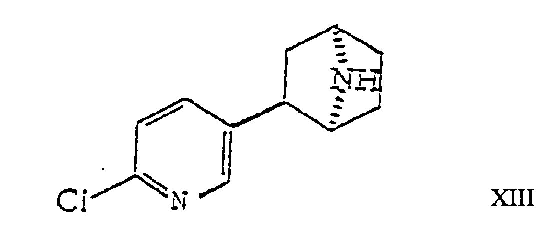 und anschließend
β) Unterziehen der Letzteren einer Epimerisierung in Gegenwart einer Base
oder
b) Cyclisieren von 1-[6'-(Chlor)-pyrid-3'-yl]-3-[oxo]-6-[nitro]-hexa-1-en der Formel 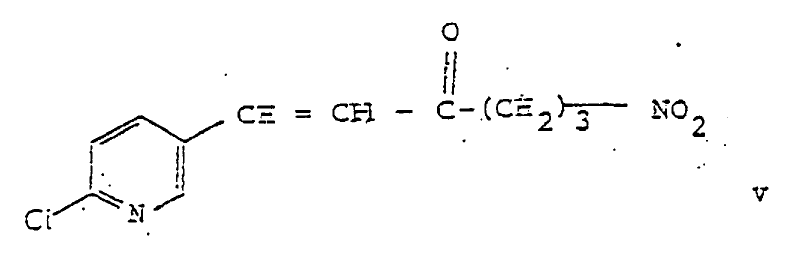 in Gegenwart einer optisch aktiven Base zu optisch aktivem 1-[Nitro]-2-[6'-(chlor)-pyrid-3'-yl]-cyclohexan-4-on der Formel 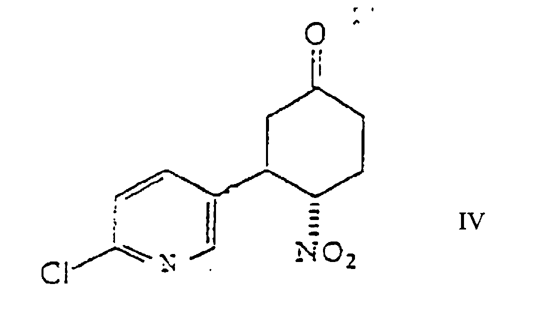 und anschließend
α) selektives Reduzieren des derart erhaltenen optisch aktiven 1-[Nitro]-2-[6'-(chlor)-pyrid-3'-yl]-cyclohexan-4-ons der Formel IV, um ein optisch aktives 1-[Nitro]-2-[6'-(chlor)-pyrid-3'-yl]-cyclohexan-4-ol folgender Formel zu erhalten 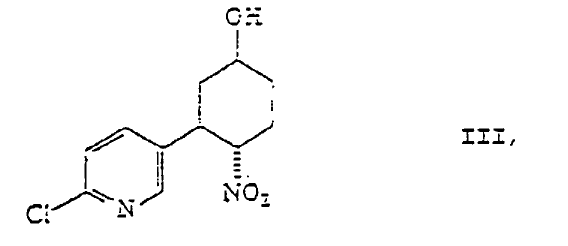
β) Einführen einer Abgangsgruppe in die Letztere, um ein optisch aktives 1-[Nitro]-2-[6'-(chlor)-pyrid-3'-yl]-cyclohexan, 4-substituiert durch eine Abgangsgruppe, der allgemeinen Formel 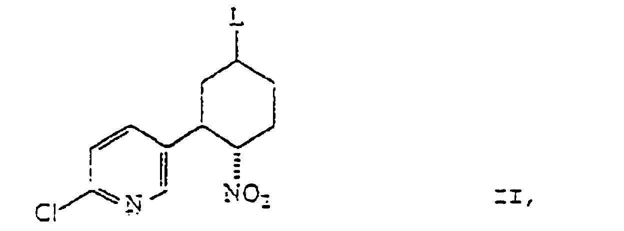 worin
L für eine Abgangsgruppe, insbesondere eine C₁₋₄-Alkylsulfonyloxy- oder Arylsulfonyloxygruppe, steht, zu erhalten,
γ) Reduzieren der Letzteren zu einem optisch aktiven 1-[Amino]-2-[6'-(chlor)-pyrid-3'-yl]-cyclohexan, 4-substituiert durch eine Abgangsgruppe, der allgemeinen Formel 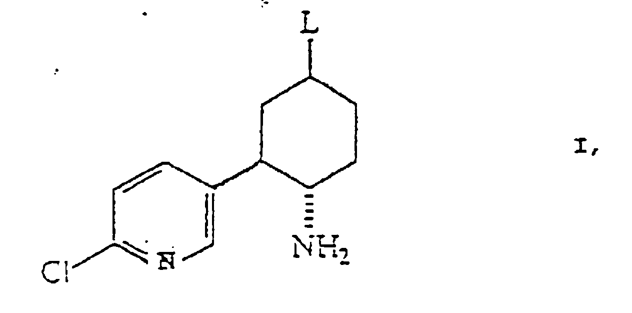
δ) Cyclisieren der Letzteren zu einem optisch aktiven epi-Epibatidin der Formel XIII
und
ε) Unterziehen der Letzteren einer Epimerisierung in Gegenwart einer Base.

2. Verfahren gemäß Anspruch 1a)β), gekennzeichnet durch die Durchführung der Epimerisierung bei einer erhöhten Temperatur.

3. Verfahren gemäß Anspruch 1a)β) oder 2, gekennzeichnet durch die Verwendung eines Alkalialkoholats oder einer anderen organischen Alkaliverbindung für die Epimerisierung.

4. Verfahren gemäß Anspruch 1a)β), 2 oder 3, gekennzeichnet durch die Verwendung von Kalium-tert.-butylat, Natriumethylat oder Lithiumdiisopropylamin als Base für die Epimerisierung.

5. Verfahren gemäß Anspruch 1a)β) oder 2 bis 4, gekennzeichnet durch die Durchführung der Epimerisierung in Gegenwart eines Alkalialkoholats unter Erwärmung und der Epimerisierung in Gegenwart einer anderen organischen Alkaliverbindung bei einer Temperatur von etwa 0 °C.

6. Verfahren gemäß Anspruch 1a)β) oder 2 bis 5, gekennzeichnet durch die Durchführung der Epimerisierung in einem C₁₋₄-Alkanol als Medium in Gegenwart eines Alkalialkoholats, das dem als Lösungsmittel verwendeten Alkanol entspricht.

7. Verfahren gemäß Anspruch 1a), gekennzeichnet durch das Cyclisieren eines 1-[Amino]-2-[6'-(chlor)-pyrid-3'-yl]-cyclohexans, 4-substituiert durch eine Abgangsgruppe, der allgemeinen Formel I, in der die durch L repräsentierte Abgangsgruppe eine Methansulfonyloxy-, p-Toluolsulfonyloxy- oder p-(Brom)-phenylsulfonyloxygruppe ist.

8. Verfahren gemäß Anspruch 1a) oder 7, gekennzeichnet durch das Cyclisieren des 1-[Amino]-2-[6'-(chlor)-pyrid-3'-yl]-cyclohexans, 4-substituiert durch eine Abgangsgruppe, der allgemeinen Formel I bei einer erhöhten Temperatur, insbesondere beim Siedepunkt der Reaktionsmischung.

9. Verfahren gemäß Anspruch 1a), 7 oder 8, gekennzeichnet durch die Durchführung der Cyclisierung in einem aprotischen Lösungsmittel, insbesondere in einem halogenierten Kohlenwasserstoff oder einem aromatischen Kohlenwasserstoff oder einer Mischung davon.

10. Verfahren gemäß Anspruch 1a) oder 7 bis 9, gekennzeichnet durch die Durchführung der Cyclisierung in Benzol, Toluol und/oder Xylol als aprotische(s) Lösungsmittel.

11. Verfahren gemäß Anspruch 1b), gekennzeichnet durch die Verwendung von (+)-α-(Phenyl)-etyhlamin oder (-)-α-(Phenyl)-etyhlamin als optisch aktive Base für die Cyclisierung.

12. Verfahren gemäß Anspruch 1, gekennzeichnet durch das Auflösung des racemischen 1-[Nitro]-2-[6'-(chlor)-pyrid-3'-yl]-cyclohexan-4-ols der Formel III und anschließendes Umwandeln des optisch aktiven 1-[Nitro]-2-[6'-(chlor)-pyrid-3'-yl]-cyclohexan-4-ols der Formel III in optisch aktives Epibatidin der Formel XIV.

13. Verfahren gemäß Anspruch 1 oder 12, gekennzeichnet durch die Durchführung der Auflösung des racemischen 1-[Nitro]-2-[6'-(chlor)-pyrid-3'-yl]-cyclohexan-4-ols der Formel III, indem es mit optisch aktivem Menthylchlorformiat verestert wird, die derart erhaltenen Diastereomere durch Kristallisation getrennt werden und anschließend die Menthylgruppe entfernt wird.

## Revendications

1. Procédé pour préparer de l'épibatidine racémique ou optiquement active de la formule 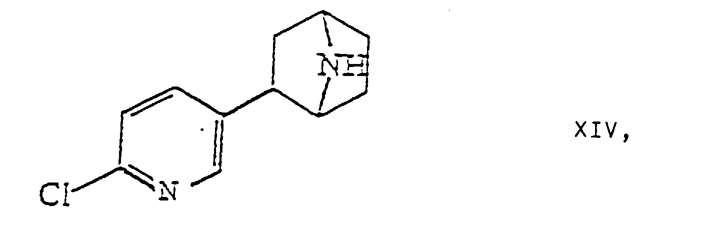 caractérisé par
a)
α) la cyclisation d'un 1-[amino]-2-[6'-(chloro)-pyrid-3'-yl]-cyclohexane, 4-substitué racémique ou optiquement actif par un groupe séparé, de la formule générale, 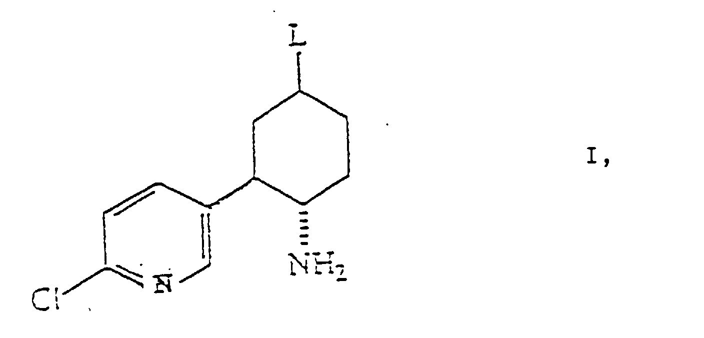 dans laquelle
L représente un groupe séparé, notamment un groupe C₁₋₄-alkylsulfonyloxy ou arylsulfonyloxy
en une épi-épibatidine racémique ou optiquement active de la formule 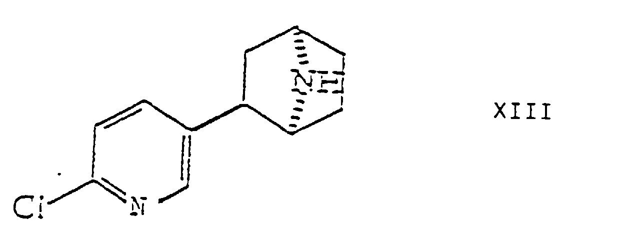 et ensuite
β) la soumission de cette dernière à l'épimérisation en présence d'une base ou
b) la cyclisation de 1-[6'-(chloro)-pyrid-3'-yl]-3-[oxo]-6-[nitro]-hexa-1-ène de la formule 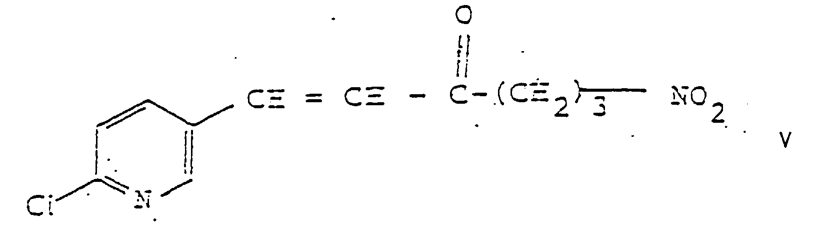 en présence d'une base optiquement active en 1-[nitro]-2-[6'-(chloro)-pyrid-3'-yl]-cyclohexane-4-one optiquement actif de la formule 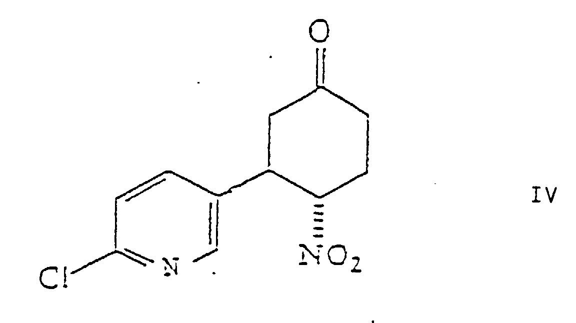
α) la réduction sélective de 1-[nitro]-2-[6'-(chloro)-pyrid-3'-yl]-cyclohexane-4-one optiquement actif de la formule IV ainsi obtenu pour produire un 1-[nitro]-2-[6'-(chloro)-pyrid-3'-yl]-cyclohexane-4-ol optiquement actif de la formule 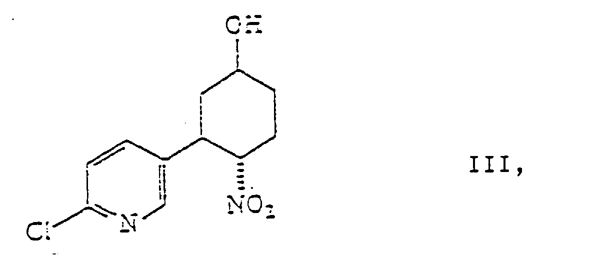
β) l'introduction dans cette dernière d'un groupe séparé pour produire un 1-[nitro]-2-[6'-(chloro)-pyrid-3'-yl]-cyclohexane, 4-substitué par un groupe séparé de la formule générale 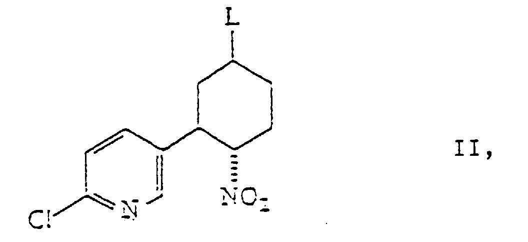 dans laquelle
L représente un groupe séparé, notamment un groupe C₁₋₄-alkyle-sulfonyloxy ou un groupe arylsulfonyloxy
γ) la réduction de cette dernière en 1-[amino]-2-[6'-(chloro)-pyrid-3'-yl]-cyclohexane, 4-substitué optiquement actif par un groupe séparé de la formule générale 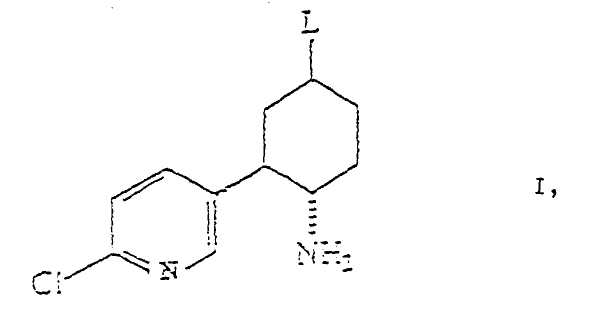
δ) la cylisation de cette dernière en une épi-épibatidine optiquement active de la formule XIII
et
ε) la soumission de cette dernière à l'épimérisation en présence d'une base.

2. Procédé selon la revendication 1a)β), caractérisé par l'exécution de l'épimérisation à une température élevée.

3. Procédé selon les revendications 1a)β) ou 2, caractérisé par l'utilisation d'un alcoolate alcalin ou d'un autre composé organique alcalin pour l'épimérisation.

4. Procédé selon les revendications 1a)β), 2 ou 3, caractérisé en ce que pour l'épimérisation un butylate tertiaire de potassium, un éthylate de sodium ou une amine de lithium diisopropyle est utilisé en tant que base.

5. Procédé selon les revendications 1a)β) ou 2 à 4, caractérisé par l'exécution de l'épimérisation en présence d'un alcoolate alcalin en chauffant et l'épimérisation en présence d'un autre composé organique alcalin à une température d'environ 0°C.

6. Procédé selon les revendications 1a)β) ou 2 à 5, caractérisé par l'exécution de l'épimérisation dans un C₁₋₄-alkanol comme milieu en présence d'un alcoolate alcalin correspondant à l'alkanol utilisé comme solvant.

7. Procédé selon la revendication 1a), caractérisé par la cyclisation de 1-[amino]-2-[6'-(chloro)-pyrid-3'-yl]-cyclohexane, 4-substitué par un groupe séparé de la formule générale I dans laquelle le groupe séparé représenté par L est un groupe méthanesulfonyloxy, p-toluènesulfonyloxy ou p-(bromo)-phenylsulfonyloxy.

8. Procédé selon les revendications 1a) ou 7, caractérisé par la cyclisation de 1-[amino]-2-[6'-(chloro)-pyrid-3'-yl]cyclohexane, 4-substitué par un groupe séparé de la formule générale I à une température élevée, notamment au point d'ébullition du mélange réactif.

9. Procédé selon les revendications 1a), 7 ou 8, caractérisé par l'exécution de la cyclisation dans un solvant aprotique, notamment dans un hydrocarbure halogéné ou un hydrocarbure aromatique ou un mélange des deux.

10. Procédé selon les revendications 1a) ou 7 à 9, caractérisé par l'exécution de la cyclisation dans du benzène, du toluène et/ou du xylène comme solvant(s) aprotique(s).

11. Procédé selon la revendication 1b), caractérisé par l'utilisation pour la cyclisation de (+)-α-(phényle)-éthyl-amine ou de (-)-α-(phényle)-éthyl-amine comme base optiquement active.

12. Procédé selon la revendication 1, caractérisé par la résolution du 1-[nitro]-2-[6'-(chloro)-pyrid-3'-yl]-cyclohexane-4-ol racémique de la formule III et ensuite la conversion du 1-[nitro]-2-[6'-(chloro)-pyrid-3'-yl]-cyclohexane-4-ol optiquement actif de la formule III dans une épibatidine optiquement active de la formule XIV.

13. Procédé selon les revendications 1 ou 12, caractérisé par l'exécution de la résolution du 1-[nitro]-2-[6'-(chloro)-pyrid-3'-yl]-cyclohexane-4-ol racémique de la formule III par l'estérification de celui-ci avec du chloroformiate de menthyle optiquement actif, par la séparation des diastéréomères obtenus par cristallisation et ensuite l'élimination du groupe de menthyle.
